# EUROPEAN PATENT APPLICATION

(11) **EP 1 574 574 A1**
(43) Date of publication of application: **14.09.2005**
(21) Application number: 03811521.8
(22) Date of filing: 14.11.2003
(51) Int. Cl.: C12N 15/12, A61K 31/7088, A61K 48/00, A61P 35/00, A61P 43/00

(54) **EXON 1-BETA OF PDGF RECEPTOR ALPHA GENE AND UTILIZATION THEREOF**

(30) Priority: 15.11.2002 JP 2002332142
(71) Applicant: Keio University, Tokyo 108-0073 (JP)
(72) Inventor: IMOTO, Masaya, c/o Keio University, Yokohama-shi, Kanagawa 223-8522 (JP); MINATO, Yusuke, c/o Keio University, Yokohama-shi, Kanagawa 223-8522 (JP); TASHIRO, Etsu, c/o Keio University, Yokohama-shi, Kanagawa 223-8522 (JP)
(74) Representative: Jones, Helen M.M.
(86) International application number: PCT/JP2003/014528
(87) International publication number: WO 2004/046356

(57) **Abstract**

By using an antisense nucleotide, a ribozyme, a maxizyme, or an RNAi constructed based on the nucleotide sequence of exon 1 β of the PDGF receptor α gene, which is expressed in specific cancer cells, or a polypeptide containing a portion thereof, translation of an mRNA transcribed from exon 1 β of the PDGF receptor α gene is suppressed. An agent for suppressing expression containing as an active ingredient a substance for inhibiting expression, such as an antisense nucleotide, a ribozyme, a maxizyme, or an RNAi, is effective as a therapeutic agent for cancer.

SEQUENCE LISTING

<110>  KEIO UNIVERSITY

<120>  EXON 1 BETA OF PDGF RECEPTOR ALPHA GENE AND USE THEREOF

<130>  HMJ04040EP

<140>  EP 03811521.8
<141>  2003-11-14

<150>  JP 02/332142
<151>  2002-11-15

<160>  8

<170>  PatentIn version 3.1

<210>  1
<211>  338
<212>  DNA
<213>  Homo sapiens

<220>
<223>  Inventor: Imoto, Masaya
       Inventor: Minato, Yusuke
       Inventor: Tashiro, Etsu

<400>  1
   gcgcgggggc gacagcggcg gcgcgggcgg gcggtctgga ataatgacaa acacatttgg      60
   ccctgagtga agaagtcgtc gtcgcctcgc attccagcaa ctgggatttg aggaatttcg     120
   aaccgcacac caaggggccc tcattgtgct ccgtggcccc cgcccccgcc cgtcttcccg     180
   cgccccctcc tcggtggaat catttctgca ttgcccgggg gctctgcttt cgctcagttc     240
   tggccgcagg caggaagaga ggaaaggtct ccaggaaggt gccgaacttc ttgtgaggaa     300
   gttagggacg acttggaact ggggaaactt gtttgcag                             338

<210>  2
<211>  363
<212>  DNA
<213>  Homo sapiens

<400>  2
cagtgtgctc gggttgcacg ccctagcgcg ggggcgacag cggcggcgcg ggcgggcggt
   60

ctggaataat gacaaacaca tttggccctg agtgaagaag tcgtcgtcgc ctcgcattcc
  120

agcaactggg atttgaggaa tttcgaaccg cacaccaagg ggccctcatt gtgctccgtg
  180

gcccccgccc ccgcctgtct tcccgcgccc cctcctcggt ggaatcattt ctgcattgcc
  240

cgggggctct gctttcgctc agttctggcc gcaggcagga agagaggaaa ggtctccagg
  300

aaggtgccga acttcttgtg aggaagttag ggacgacttg gaactgggga aacttgtttg
  360

cag
  363



<210>  3
<211>  17
<212>  DNA
<213>  Artificial Sequence

<220>
<223>  Promoter sequence

<400>  3
taatacgact cactata
   17



<210>  4
<211>  36
<212>  DNA
<213>  Artificial Sequence

<220>
<223>  Forward primer 1

<400>  4
ccttaattaa gggattctcg catgccagag atccta
   36



<210>  5
<211>  36

<212>  DNA
<213>  Artificial Sequence

<220>
<223>  Reverse primer 1

<400>  5
ccttaattaa ggggcgcaac tgcaactccg ataaat
   36



<210>  6
<211>  37
<212>  DNA
<213>  Artificial Sequence

<220>
<223>  Forward primer 2

<400>  6
ccttaattaa ggaaccgcac accaaggggc cctcatt
   37



<210>  7
<211>  23
<212>  DNA
<213>  Artificial Sequence

<220>
<223>  Reverse primer 2

<400>  7
aacagcacag gtgaccacaa tcg
   23



<210>  8
<211>  33
<212>  DNA
<213>  Artificial Sequence

<220>
<223>  Reverse primer 3

<400>  8
ccgctcgagg cgacgacgac ttcttcactc agg
   33

## Description

### CRROSS REFERENCE TO RELATED APPLICATIONS

This application claims the benefit of priority to Japan Patent Application No. 2002-332142, filed on November 15, 2002, which is incorporated herein by reference.

### TECHNICAL FIELD

The present invention relates to polynucleotides that include exon 1 β of the PDGF receptor α gene or a part thereof, to methods, substances, and agents for suppressing expression of PDGF receptor α which target mRNA including exon 1 β among mRNAs of the PDGF receptor α gene and to cancer therapeutic agents.

### BACKGROUND ART

Platelet-derived growth factor (PDGF) plays an important role in cell proliferation, development and differentiation, wound healing, malignant progression of cancer and arteriosclerosis, etc. It is therefore expected that regulation of PDGF signaling will lead to discovery of therapeutic agents for these diseases. As therapeutic agents, in fact, PDGF expression suppressors (refer to, e.g. , Japanese Laid-Open Application No. 10-59850), inhibitors of binding between PDGF and PDGF receptor α (National Publication of International Patent Application No. 1996-13370), and tyrosine kinase inhibitor of PDGF receptor α (refer to, e.g., National Publication of International Patent Application No. 2002-514228) have been proposed.

However, these suppressors and inhibitors can affect not only cancer-specific PDGF signaling but also normal PDGF signaling. Thus, the object of the present invention is to provide polynucleotides, substances for suppressing expression, agents for suppressing expression, and cancer therapeutic agents for use in methods for suppressing expression that enable selective suppression of PDGF signals specific to cancer cells.

### DISCLOSURE OF THE INVENTION

The polynucleotide according to the present invention has the nucleotide sequence shown in SEQ ID NO: 2 or a part thereof. An example of a polynucleotide having a part of the nucleotide sequence shown in SEQ ID NO: 2 is, for example, the one having the nucleotide sequence shown in SEQ ID NO: 1.

Further, the polynucleotide according to the present invention has the nucleotide sequence of SEQ ID NO: 2 with one or a few nucleotides deleted, substituted, or added, or a part thereof, which is included in the nucleotide sequence of the sense strand of the PDGF receptor α gene.

"PDGF receptor α gene" as used herein refers to the gene composed of the nucleotide sequence shown in GenBank Accession Nos. AC026580 and AC025013 and its homologues.

Further, the polynucleotide according to the present invention may be a polynucleotide that has a nucleotide sequence complementary to the aforementioned polynucleotide or part thereof.

These polynucleotides may be any one of double-stranded DNA, single-stranded DNA, double-stranded RNA, and single-stranded RNA. Further, a polypeptide that has a genetic polymorphism which is deletion, substitution, or addition of one or a few nucleotides, and that has a nucleotide sequence included in the nucleotide sequence of the sense strand of the PDGF receptor α gene or a part thereof is within the scope of the present invention. However, the polypeptide is preferred to have an equivalent function and is more preferred to be transcriptionally regulated by E2F-1.

The method for suppressing expression of PDGF receptor α according to the present invention targets mRNA containing exon 1 β among mRNAs of the PDGF receptor α gene. "mRNA" as used herein refers to RNA formed by removing the introns from hnRNA and linking the exons. "To target an mRNA" as used herein refers to specifically preventing, directly or indirectly from the mRNA, formation of its encoded protein. "Protein expression" refers to production of proteins as a result of accurate translation of genetic information on DNA through mRNA.

The method for suppressing expression of PDGF receptor α according to the present invention may be any one that uses an antisense nucleotide, a ribozyme, a maxizyme, or an RNAi.

"Antisense nucleotide of the PDGF receptor α gene" as used herein refers to a nucleotide complementary to the nucleotide sequence of mRNA of the PDGF receptor α gene. The antisense nucleotide of the PDGF receptor α gene may be an antisense RNA or an antisense DNA, and modified nucleotides may be used. The above-mentioned antisense RNA or DNA refers an RNA or a DNA complementary to a mRNA sequence transcribed from a target gene. An antisense RNA or DNA is used to block expression of genetic information in a cell and to specifically suppress production of the target protein.

"Ribozyme" is the general term of RNA with enzyme activity; it refers to an enzyme that specifically cleaves organism-based RNA. A ribozyme has the function of cleaving a target RNA sequence when taken up into cells, resulting in suppression of protein expression from the target RNA. A ribozyme is preferable as a substance for suppressing protein expression because of its high specificity to a target RNA sequence. Ribozymes include a hammerhead ribozyme, hairpin ribozyme, etc.

"Maxizyme" is generally RNA molecules that form the dimer structure as described in WO99/46388. For example, it is possible to cleave only cancer cell-specific mRNAs by constructing a maxizyme so that its two RNA molecules recognize cancer cell-specific mRNAs, instead of recognizing mRNA in a normal cell.

"RNAi" is a technique using double-stranded RNA (dsRNA) that induces a phenomenon called RNA interference. "Phenomenon called RNA interference" refers to a phenomenon in which expression of a target gene is suppressed when the double-stranded RNA is introduced into a cell. Currently, RNAi is considered to work like this: an endogenous mechanism in a host cuts an RNA molecule into 21- 23 base-pair short RNAs. These short RNA molecules recognize and sequence-specifically degrade mRNA transcribed from a host gene. As a result, expression of the protein encoded by the host gene is specifically suppressed.

The method for suppressing expression according to the present invention may be any one that uses DNA that encodes an antisense RNA, a ribozyme, a maxizyme, or an RNAi.

The substance for suppressing expression of PDGF receptor α according to the present invention targets a mRNA containing exon 1 β among mRNAs of the PDGF receptor α gene. This is caused, for example, by binding of a substance for suppressing expression to the mRNA or its degradation of the mRNA. Furthermore, a substance for suppressing expression may indirectly cause some other substance to bind to the mRNA or to degrade the mRNA. It should be noted that "substance for suppressing expression of PDGF" refers to a substance that suppresses production of the PDGF receptor α protein from the PDGF receptor α gene.

Specific examples of the substance for suppressing expression of PDGF receptor α according to the present invention include an antisense nucleotide, a ribozyme, a maxizyme, or an RNAi.

Specific examples of the substance for suppressing expression of PDGF receptor α according to the present invention include DNA that encodes antisense RNA, a ribozyme, a maxizyme, or an RNAi.

The agent for suppressing expression of PDGF receptor α according to the present invention contains the aforementioned substance for suppressing expression as an active ingredient.

The therapeutic agent for cancer according to the present invention contains the aforementioned agent for suppressing expression.

The therapeutic method for cancer according to the present invention uses the aforementioned agent for suppressing expression.

### BRIEF DESCRIPTION OF THE DRAWINGS

FIG. 1 shows the expression patterns of exons 1 β to 4 of the PDGF receptor α gene, examined using RT-PCR in Example 2 of the present invention.
FIG. 2 shows the structure in a ribozyme.
FIG. 3 shows the structure in a maxizyme.
FIG. 4 shows a result of luciferase assay using a reporter construct including the sequence from nucleotides -1395 to +312 in Example 1 according to the present invention. "+" in the figure shows a result of introduction of 100 ng of the reporter construct including the sequence from nucleotides 1395 to +312. "-"in the figure shows a result of introduction of 100 ng of the reporter construct lacking the sequence from nucleotides -1395 to +312.
FIG. 5 shows a result of luciferase assay using a reporter construct including the sequence from nucleotides -517 to +1445 in Example 1 according to the present invention.
FIG. 6 shows a result of luciferase assay using deletion mutants of various lengths that lack their transcription start points in Example 1 of the present invention.
FIG. 7 schematically shows mRNA of PDGFRα transcribed by basic transcription factors and mRNA of PDGFRα transcribed by E2F-1.

### BEST MODE FOR CARRYING OUT THE INVENTION

It is known that in cancer cells, in most cases, abnormalities have occurred in the signal transduction pathway in which cancer suppressor proteins, such as the RB protein, are involved. For example, overexpression of cycline D1, which functions upstream of the RB protein, is considered to contribute to malignant progression of cancer cells by enhancing sensitivity to growth factors. In an in vitro cell culture system, by adding fibroblast growth factor (FGF) to a cell line in which cycline D1 is overexpressed, the cells become malignant. The inventors found out that platelet-derived growth factor (PDGF) also functions in the same manner--i.e., cause a cell line in which cycline D1 is overexpressed to become malignant.

The transcription factor E2F-1 is present downstream in the cycline D1-RB pathway and enhances its sensitivity to FGF by enhancing expression of FGF receptors. E2F-1 enhances expression of PDGF receptor α as well but the inventors found that E2F-1 does not act on the known promoter. The inventors found out a novel promoter region regulated by E2F-1 in the conventional intron 1 and identified novel exon 1 β to be used in transcription by the novel promoter, as will be described in detail in Examples.

Thus, it has been shown that, in the malignant progression of cancer involving E2F-1 and PDGF, one of the targets of these factors is mRNA that contains exon 1 β of PDGF receptor α. Consequently, by specifically inhibiting production of PDGF receptor α from transcripts having this exon 1 β, it is possible to block the signaling pathway leading to malignant progression of cancer cells and thereby to suppress proliferation of cancer cells. It should be noted that, when proliferation of cancer cells is induced by some factor that cause the cancer to be malignant in collaboration with PDGF receptor α by inducing overtranscription of mRNA containing exon 1 β, the present invention is applicable to inhibition of proliferation of such cancer cells, even if the cycline D1-E2F-1 pathway is not involved.

Embodiments of the present invention accomplished based on the above-described findings are hereinafter described in detail by giving Examples. Unless otherwise explained, methods described in standard sets of protocols such as J.Sambrook and E.F.Fritsch & T.Maniatis (Ed.),"Molecular Cloning, a Laboratory Manual (2nd edition), Cold Spring HarborPress and Cold Spring Harbor, New York (1989); and F.M.Ausubel, R.Brent, R.E.Kingston, D.D.Moore, J.G.Seidman, J.A.Smith, and K.Struhl (Ed.),"Current Protocols in Molecular Biology," John Wiley & Sons Ltd., or alternatively, their modified/changed methods are used. When using commercial reagent kits and measuring apparatus, unless otherwise explained, protocols attached to them are used.

The object, characteristics, and advantages of the present invention as well as the idea thereof will be apparent to those skilled in the art from the descriptions given herein. It is to be understood that the embodiments and specific examples of the invention described herein below are to be taken as preferred examples of the present invention. These descriptions are only for illustrative and explanatory purposes and are not intended to limit the invention to these embodiments or examples. It is further apparent to those skilled in the art that various changes and modifications may be made based on the descriptions given herein within the intent and scope of the present invention disclosed herein.

A polynucleotide having the nucleotide sequence of the exon 1 β of the human PDGF receptor α gene shown in SEQ ID NO: 2 or a part thereof can be prepared, based on nucleotide sequence information shown in SEQ ID NO: 2, from human a gene library, such as a cDNA library or a genomic library. In addition, exon 1 β of the PDGF receptor α gene derived from organisms other than humans, such as mice, rats, chicks, pigs, dogs, and monkeys can be also defined by being transcriptionally regulated by E2F-1. It can also be identified, for example, by hybridization to exon 1 β of the human PDGF receptor α gene or examination of a new exon in the conventionally known intron 1 sequence. The exon 1 β of the PDGF receptor α gene derived from organisms other than humans is also among the polynucleotides according to the present invention.

"Exon 1 β of the human PDGF receptor α gene" as used herein refers to the exon including the polynucleotide that has the nucleotide sequence shown in SEQ ID NO: 2. " Exon 1 β of the PDGF receptor α gene" refers to the exon containing the polynucleotide that has the nucleotide sequence shown in SEQ ID NO: 2 and the exon corresponding thereto in species other than humans.

Being the non-coding region on mRNA, exon 1 β of the PDGF receptor α gene does not necessarily need to have a high homology at the nucleotide sequence level in species other than humans, but it needs to conserve the functional aspect of being transcribed in a specific cell type; for example, it needs to have the feature that its transcription is regulated by E2F-1, which another exon 1 does not have.

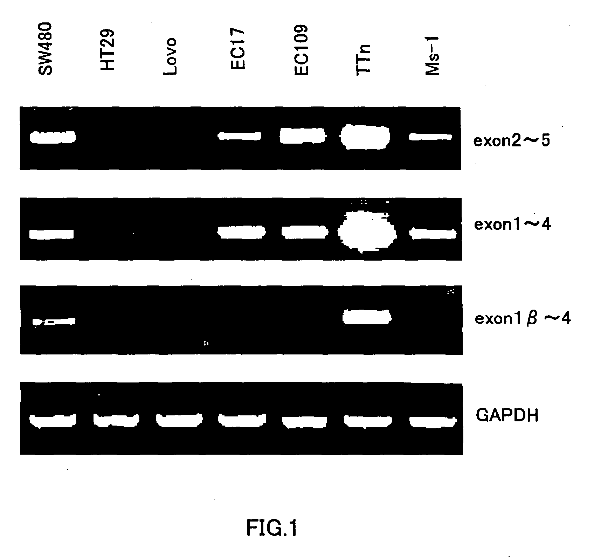

As shown in FIG. 1, mRNA containing exon 1 β among mRNAs of the PDGF receptor α gene is detected only in specific cancer cells. Therefore, the method for suppressing expression that targets exon 1 β-containing mRNA among mRNAs of the PDGF receptor α gene, the polynucleotide, the substance for suppressing expression, the agent for suppressing expression, etc. according to the present invention are useful as therapeutic methods and agents for cancer for the purpose of attacking specific cancer cells (illustratively, e.g., human colon cancer cells SW480, human esophageal cancer cells T.Tn, etc.)

The method for suppressing expression according to the present invention targets mRNA containing exon 1 β among mRNAs of the PDGF receptor α gene. It may be, for example, the method for suppressing expression of PDGF receptor α by causing the mRNA containing exon 1 β among mRNAs of a PDGF receptor α gene to be bound, thereby suppressing translation. Alternatively, it may be the method for suppressing expression of PDGF receptor α by degrading or cleaving the above-mentioned mRNA. In the following, the methods for suppressing expression using substances for suppressing expressions, such as an antisense nucleotides, a ribozyme, a maxizyme, or an RNAi, will be described by means of examples.

### (1) PREPARATION OF ANTISENSE NUCLEOTIDES

The antisense nucleotides for use in the method for suppressing expression according to the present invention are illustratively antisense RNA or antisense DNA having the nucleotide sequence complementary to the nucleotide sequence of the portion corresponding to exon 1 β among mRNAs of the PDGF the nucleotide sequence complementary to part thereof. In this case, an antisense oligonucleotide consisting of a 15-30 nucleotide sequence may be used.

The antisense nucleotide according to the present invention is not limited, in terms of structure, to the location or length of its sequence, modifications, or presence of mismatches in the sequence. The above-mentioned modified antisense nucleotides are illustratively the antisense nucleotide linked by phosphodiester bonds that have a phosphate group with one oxygen atom modified with a sulfur atom or a methyl group for stabilization of antisense nucleotides in the body or cells or the morpholino-modified antisense nucleotide. 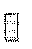

The antisense DNA according to the present invention can be synthesized in vitro by the primer extension method using a DNA-dependent DNA polymerase such as S1 nuclease. It is also possible to synthesize only antisense strands by performing PCR using a part of the antisense strand as primers. Alternatively, antisense DNA may be synthesized artificially by using a DNA synthesizer or other means. Antisense oligonucleotides are synthesized in the same manner as antisense DNA and are most preferably artificially synthesized.

On the other hand, the antisense RNA according to the present invention can easily be synthesized with an RNA synthesizer, by solid-phase synthesis, or other means. Subsequently, target RNA can be isolated by elution with NH3OH/EtOH using HPLC.

The antisense RNA according to the present invention may be artificially synthesized as described above, but it may be synthesized in an in vitro transcription system with T7 RNA polymerase by constructing a vector into which a double-stranded DNA having a DNA sequence corresponding to antisense RNA has been inserted downstream of the promoter sequence (5'-TAATACGACTCACTATA-3': SEQ ID NO: 3) specifically recognized by T7 RNA polymerase.

Alternatively, antisense RNA may be expressed in cells using expression vectors, such as a virus vector or a plasmid incorporating DNA corresponding to the antisense RNA. The virus vector may be an adenovirus vector or a retroviral vector.

### (2) PREPARATION OF A PLASMID INTO WHICH A RIBOZYME HAS BEEN INSERTED

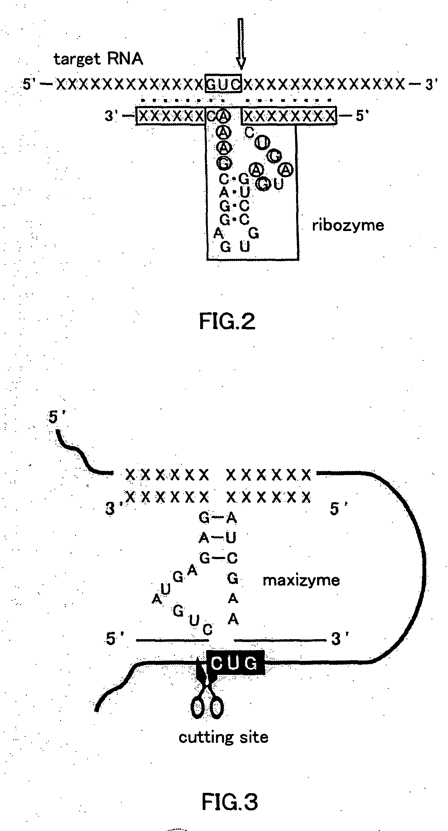

A ribozyme has nucleotide sequences (sequences at the 5'and 3' ends of a ribozyme are shown) complementary to the target mRNA sequences and a 24-nucleotide sequence including the catalytic active site (circled nucleotides), as shown in FIG. 2. When the 5' and 3' end sequences of a ribozyme specifically bind to the target mRNA sequence, it is possible to cleave mRNA at sequence NUX (where N can be any nucleotide and X can be A, U or C) and thereby to degrade the target mRNA. Accordingly, it is considered that by synthesizing a ribozyme using as a substrate the nucleotide sequence of the portion corresponding to exon 1 β among mRNAs of the PDGF receptor α gene and administering such a ribozyme into cells, it is possible to specifically cleave mRNAs containing Exon 1 β among mRNAs of the PDGF receptor α gene.

Therefore, the ribozyme that is the substance for suppressing expression according to the present invention can be designed such that, after selecting the sequence corresponding to an NUX sequence from the nucleotide sequence of exon 1 β of the human PDGF receptor α gene shown in SEQ ID NO: 2, both a nucleotide sequence complementary to a 15-20 nucleotide sequence containing the selected sequence and a 24-nucleotide sequence including the catalytic active site (circled nucleotides) are included in the ribozyme. Specific examples of the sequence corresponding to an NUX sequence include, for example, GTCs at positions 34 to 36, GTCs at positions 75 to 77, GTCs at positions 78 to 80, GTCs at positions 81 to 83, GTCs at positions 172 to 174, and GTCs at positions 267 to 269 shown in SEQ ID NO: 2. The above-mentioned ribozyme may be a hammerhead ribozyme and a hairpin ribozyme.

The actual methods for synthesizing the ribozyme include artificial synthesis, in vitro synthesis, intracellular synthesis with an expression vector, etc., and as the techniques are the same as those used for antisense RNA synthesis as described in (1), their explanation is omitted here.

### (3) DESIGN AND PREPARATION OF A MAXIZYME

As shown in FIG. 3, a maxizyme is composed of RNA molecules that form the dimer structure. Each of the two RNA molecules has a sensor arm (X...X region in the figure, X representing any nucleotide. X in the upper row and the corresponding X in the lower row represent a pair of complementary nucleotides.) that specifically recognizes a target RNA; a catalytic active site (the region where double strands are not formed in the figure); and a site that recognizes an NUX sequence (where N can be any nucleotide and X can be A, U or C; represented as the sequence GUC in the figure) on the mRNA containing the target RNA and upstream (upstream of the sequence GUC in the figure) or downstream (downstream of the sequence GUC portion in the figure) thereof. When a sensor arm of a maxizyme has specifically recognized and bound to the target RNA, and another sensor arm of the maxizyme has specifically recognized and bound to the upstream and downstream of an NUX sequence on the mRNA containing the target RNA, the maxizyme can cleave the NUX sequence on the mRNA containing the target RNA, thereby degrading the target mRNA. Accordingly, it is considered that by synthesizing a maxizyme on the base of the nucleotide sequence of the portion corresponding to exon 1 β in mRNA of the PDGF receptor α gene and administering such a maxizyme into cells, it should be possible to specifically cleave mRNA containing exon 1 β among mRNAs of the PDGF receptor α gene.

For example, exon 1 β of the PDGF receptor α gene is used as the target RNA and a nucleotide sequence complementary to this target RNA as a sensor arm. An NUX sequence that is present downstream of the target RNA is then selected from mRNA of PDGF receptor α and the nucleotide sequence complementary to the sequences upstream and downstream of the NUX sequence is determined. The NUX sequence may be the one on mRNA of exon 1 β of the PDGF receptor α gene. Based on these pieces of information, a maxizyme can be prepared by synthesizing each RNA molecule of the intended maxizyme with an RNA synthesizer. There may be one or a few increases or decrease in the number of the Xs shown in the figure.

The actual methods for synthesizing the ribozyme include artificial synthesis, in vitro synthesis, intracellular synthesis with an expression vector, etc. and as the techniques are the same as those used for antisense RNA synthesis as described in (1), their explanation is omitted here. It should be noted that, since a maxizyme uses two RNA molecules, when a maxizyme is expressed in cells by means of expression vectors, two RNA molecules may be incorporated into one vector or each RNA molecule may be separately incorporated into two vectors.

### (4) PREPARATION OF RNAi

It was reported that introduction of double-stranded RNA corresponding to a gene of interest into an organism causes degradation of the corresponding mRNA (Bass, B. L. (2000) Cell 101, 235-238, Fire, A.(1999) Trends Genet.15, 358-363, Sharp, P.A.(2001) Genes Dev.15 485-490). It is therefore considered that, when double-stranded RNA (RNAi) corresponding to the mRNA containing exon 1 β of the PDGF receptor α gene or a part thereof is introduced into cells or an organism, mRNA containing exon 1 β among mRNAs of the PDGF receptor α gene is degraded.

RNAi that is a substance for suppressing expression according to the present invention can be prepared as follows. RNA having the nucleotide sequence of the sense strand of exon 1 β of the PDGF receptor α gene or a part thereof and RNA complementary to the RNA can be artificially synthesized using an RNA synthesizer, or may be synthesized in vitro and in vivo using HiScribeRNAi Transcription Kit (manufactured by NEB).

Alternatively, by introducing, into cells, expression vectors, such as virus vectors or plasmids, into each of which exon 1 β of the PDGF receptor α gene or a part thereof has been cloned in positive or negative direction, and expressing both strands of DNA in cells, RNAi is formed in the cells and the target mRNA is degraded. An expression vector can be used including DNA having a sequence which has resulted from fusion of the DNA sequences of each strand of the double strand corresponding to exon 1 β of the PDGF receptor α gene or a part thereof, i.e. , DNA having a sequence in which the 3' end of the sense strand DNA has been fused to the 5' end of the antisense strand DNA or DNA having a sequence in which the 3' end of the complementary DNA has been fused to the 5' end of the sense strand DNA. The above-mentioned virus vector may be an adenovirus vector, a retroviral vector, or the like. These RNAis are preferably of up to 30 bases long, most preferably, of up to 21 bases long.

### (5) INTRODUCTION OF A SUBSTANCE FOR SUPPRESSING EXPRESSION

In a in vitro cell culture system, for intracellular introduction of a substance for suppressing expression, a prepared substance for suppressing expressions, such as an antisense nucleotide, a ribozyme, a maxizyme, or an RNAi, is introduced into the intended cancer cells by the electroporation method, microinjection method, lipofection method, viral infection method using a viral vector (e.g., an adenovirus or a retrovirus), transfection method using calcium, or the like.

On the other hand, as the method for introducing an agent for suppressing expression to an individual in vivo, an agent for suppressing expression that contains as an active ingredient a substance for suppressing expressions, such as the aforementioned prepared antisense nucleotide, ribozyme, maxizyme, or RNAi may be directly administered to the vicinity of targeted cancer cells in a human or a vertebrate other than a human. Alternatively, depending on the agent, parenteral, oral, intradermal, subcutaneous, intravenous, intramuscular, or intraperitoneal administration may be performed. In this case, an agent for suppressing expression may further contain a suitable pharmacologically acceptable excipient or base, depending on the site or purpose of administration.

An agent for suppressing expression to be administered to an individual is preferably prepared such that the substance for suppressing expression is easily taken up into cells. As one possible method, for example, an expression vector made by integrating the aforementioned antisense nucleotide, ribozyme, maxizyme, or RNAi into a viral vector is infected into a suitable cell line in vitro so that it produces the virus, and the produced virus can be used for infection by injection. The virus vector to be used may be an adenovirus vector or a retroviral vector which can function in cells.

In addition, a plasmid may be introduced into cells by encapsulating the aforementioned expression vector in a liposome so that it fuses to cancer cells. Alternatively, in vivo transfection may be performed using TransIT In Vivo Gene Delivery System (TAKARA). In this case, the agent for suppressing expression may be directly injected into the affected site or intravenously injected.

Alternatively, an RNA aptamer in which the aforementioned antisense nucleotide, ribozyme, a maxizyme, or RNA such as RNAi has been bound to peptides, such as HIV TAT, which are easily introduced into cells, may be injected as an agent for suppressing expression by an in vitro selection method. Cancer cells to be targeted are illustratively human SW 480 colon cancer cells or human T.Tn esophageal cancer cells, but they are not limited to any specific ones, as long as they are cancer cells in which mRNA transcribed from exon 1 β of the PDGF receptor α gene can be detected.

### (6) ASESSMENT OF SUPPRESSION OF EXPRESSION BY A SUBSTANCE FOR SUPPRESSING EXPRESSION

According to the methods described in the previous (5), by preparing specific cancer cells cultured in vitro with or without a substance for suppressing expression and by comparing and evaluating the amount of mRNA transcribed from exon 1 β of the PDGF receptor α gene by the RT-PCR method, suppression of expression by a substance for suppressing expression can be assessed. Alternatively, a method for assessing the amount of mRNA transcribed from exon 1 β of the PDGF receptor α gene by northern blotting etc. may be used. Based on these results, antisense nucleotides capable of suppressing more effectively translation of mRNA transcribed from exon 1 β of the PDGF receptor α gene can be found.

Although the above-mentioned procedure is for in vitro experiment, it is possible to make an assessment in in vivo experiment as well.

The in vivo assessment method is illustratively the following : The aforementioned specific cancer cells are subcutaneously injected into normal mice, the tumor is allowed to grow during a particular period of time, and subsequently, the aforementioned agent for suppressing expression prepared by the method described (5) is injected as single or multiple doses. Following injection(s), suppression of expression by the substance for suppressing expression can be assessed by comparing and evaluating tumor sizes and survival rates between the treated mice and non-treated mice.

Examples according to the present invention will be described in detail hereinbelow.

### EXAMPLE 1

In this example, the novel exon regulated by the transcription factor E2F-1 in the PDGF receptor α gene was identified.

First, the inventor found out that mouse NIH 3T3 cells proliferate by addition of PDGF, in a manner not depending on the scaffold. Thus, expression of PDGF receptor α (PDGFR-α) in the mouse NIH 3T3 cell line was examined and it was found that the expression of PDGF receptor α was enhanced, being regulated at the transcriptional level.

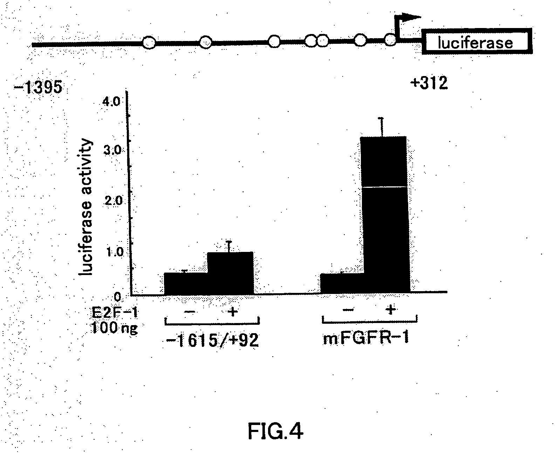

Since cycline D1 activates the transcription factor E2F-1 through cell cycle-dependent pRb phosphorylation, it was investigated whether or not this enhancement was due to the regulation of the promoter of human PDGF receptor α by E2F-1. The promoter region consisting of sequences from -1395 to +312 (nucleotides numbered according to the transcription start point reported in Genomics, Vol.30, 224-232, 1995. Refer to GenBank Accession No. D50001S01) relative to the transcription start point was cloned upstream of a luciferase gene using the pGL3 luciferase vector and introduced into cycline D1-overexpressed NIH 3T3 cells together with an E2F-1 expression vector by transfection. After culture for 24 to 72 hours, luciferase assay was performed to examine transcriptional activity by the above-mentioned promoter. As a positive control, a plasmid containing a luciferase gene downstream of the mFGFR-1 promoter was used. As a result, transcriptional activity of the mFGFR-1 promoter was enhanced, whereas transcriptional activity of the PDGF receptor α promoter was not enhanced (FIG. 4). These results revealed that the conventionally known PDGF receptor α promoter is not involved in enhancement of expression of the PDGF receptor α in mouse cycline D1-overexpressed NIH 3T3 cells.

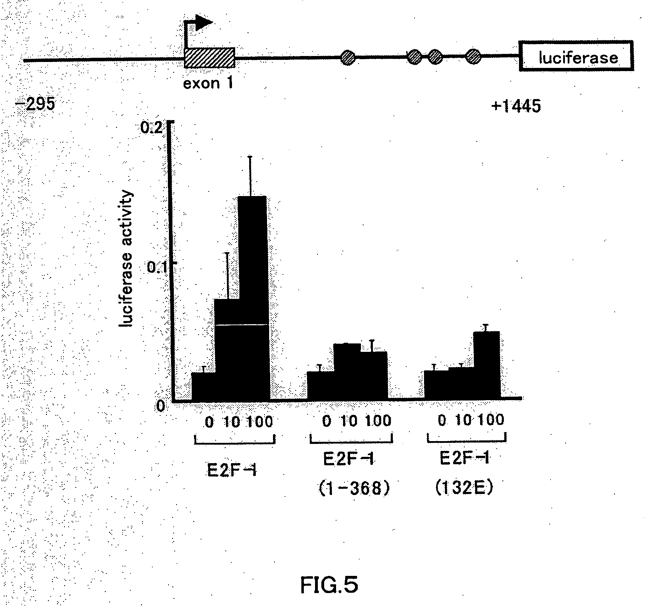

Then, it was examined whether or not a consensus sequence that binds to E2F-1 is present downstream of exon 1 of the human PDGFR-α gene by the search for transcription factor binding sequences (TF search). It was found that sequences to which E2F-1 would bind are present at four locations in clusters near approximately 1 kbp downstream of the reported transcription start point. Thus, a DNA consisting of the sequence from nucleotides -295 to +1445 was inserted upstream of a luciferase gene. By using the obtained reporter construct, luciferase assay was performed in the same manner previously described to examine transcriptional activity by E2F-1. The result confirmed that the transcriptional activity in this region is enhanced by E2F-1 (FIG. 5). Further, no transcription activity was detected with an E2F-1 mutant (amino acid sequence from 1 to 368) defective in E2F-1 transcription activation or another E2F-1 mutant defective in DNA binding ability constructed by substituting leucine 132 for glutamic acid, suggesting that the region is in fact regulated by E2F-1.

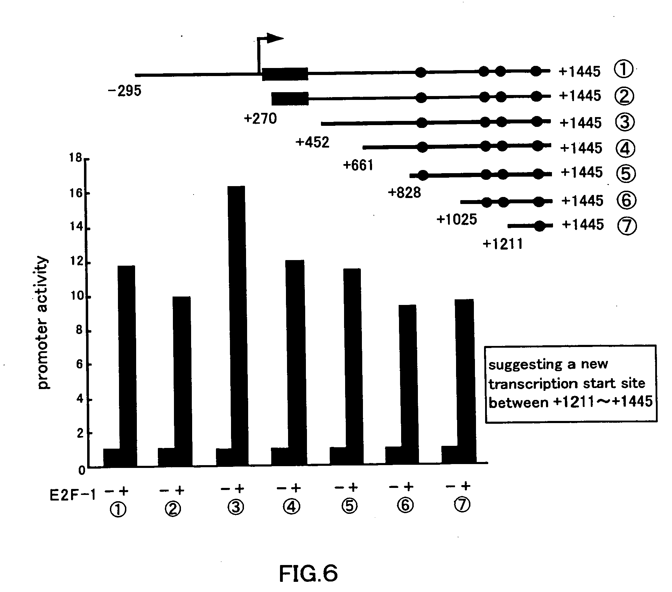

However, since the putative E2F-1 binding site is present about 1.2 kbp downstream of the transcription start point previously reported, this promoter activity is unlikely to act on the transcription start point previously reported. Hence, to examine whether the region is working on the transcription start point previously reported, deletion mutants of various lengths lacking this transcription start point were constructed and luciferase assay was performed in the same manner as above described to examine transcriptional activity by E2 F-1. As a result, even the construct deleted to about 1 kbp downstream of the transcription start point had promoter activity and further exhibited enhancement of transcriptional activity by E2F-1 as well. These results suggested a possibility that mRNA regulated by E2F-1 of PDGFR-α might be expressed at a new transcription start point (FIG. 6).

Then, the transcription start point associated with E2F-1 was determined by the 5'RACE method. mRNA was extracted from the NIH 3T3 cell line into which the reporter construct in which DNA having the sequences from nucleotides -295 to +1445 was inserted upstream of a luciferase gene had been introduced by transfection. PCR was performed with primers (forward primer 1 [SEQ ID NO: 4:5'-CCT TAATTAAGGGATTCTCGCATGCCAGAGATCCTA-3']; reverse primer 1 [SEQ ID NO: 5:5'-CCTTAATTAAGGGGCGCAACTGCAACTCCGATAAA T-3'])specific to the luciferase gene sequence by using 5'Full RACE Core Set (TAKARA's brand name), yielding amplified products. Examination of the DNA sequences of these amplified products revealed the presence of a novel exon in a region previously regarded as an intron. Thus, it was shown that a novel exon transcriptionally regulated by E2F-1 is present in a region previously considered an intron.

### EXAMPLE 2

In this Example, it was examined if exon 1 β of the PDGF receptor α gene is specifically expressed in cancer cells.

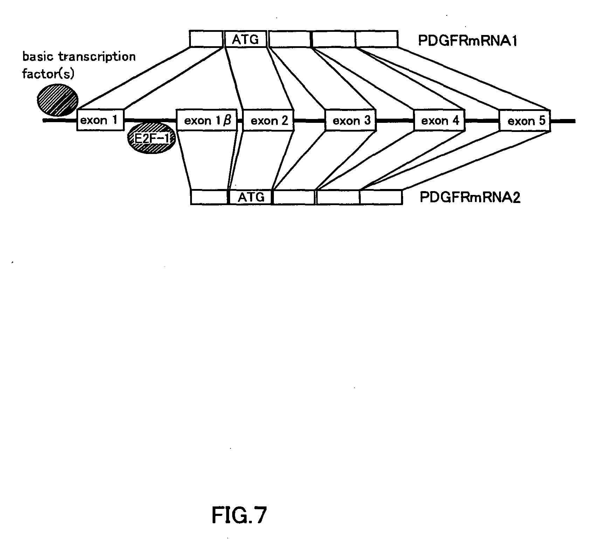

Primers (forward primer 2 [SEQ ID NO: 6:5'-CCTTAATTAAGGAACCGCACACCAAGGGGCCCTCATT-3'); reverse primer 2 [SEQ ID NO: 7:5'-AACAGCACAGGTGACCACAATCG-3']) were designed from the sequences of exon 1 β of the PDGF receptor α gene and the previously known exon 4. The RT-PCR was performed using the total RNAs extracted from various cancer cells shown in FIG. 1. As shown in FIG. 1, signals were detected in SW480 human colon cancer cells and human T. Tn oesophageal cancer cells. These amplified bands were recovered and the DNA sequences were examined. The results revealed that these bands were mRNA fragments of human PDGFR-α in which the sequences of exon 1 β of the PDGF receptor α gene and the previously known exons 2 to 4 are linked together. Simultaneously, the 338 bp full-length sequence of exon 1 β shown in SEQ ID NO: 1 was determined. Namely, the newly identified promoter region was revealed to belong to Human PDGFR mRNA2 (FIG. 7).

### EXAMPLE 3

More exact location of 5' end of exon 1 β was examined by 5'RACE method. mRNA was extracted from human MG-63 osteosarcoma cells expressing PDGFR-α mRNA including exon 1 β . By using 5'Full RACE Core Set, PCR was performed with primers (forward primer 2 [SEQ ID NO: 6]; reverse primer 3 [SEQ ID NO: 8: 5'-CCGCTCGAGGCGACGACGACTTCTTCACTCAGG-3'] specific to exon 1 β. DNA sequencing of amplified products obtained by this PCR revealed that the 363 bp nucleotide sequence shown in SEQ ID NO: 2 had been obtained and 5' end of exon 1 β extended to at least +1210.

### INDUSTRIAL APPLICABILITY

As described above, according to the present invention, polynucleotides, substances for suppressing expression, agents for suppressing expression, and cancer therapeutic agents, for use in methods for suppressing expression that enable selective suppression of PDGF signals specific to cancer cells, can be provided.

## Claims

1. A polynucleotide comprising the nucleotide sequence shown in SEQ ID NO: 2 or a part thereof.

2. A polynucleotide comprising a nucleotide sequence shown in SEQ ID NO: 2, in which one or a few nucleotides are deleted, substituted, or added, comprising a nucleotide sequence contained in the nucleotide sequence of the sense strand of the PDGF receptor α gene or a part thereof.

3. A polynucleotide comprising a nucleotide sequence complementary to the polynucleotide or part thereof of claim 1 or 2.

4. A method for suppressing expression of PDGF receptor α comprising targeting mRNA including exon 1 β among mRNAs of the PDGF receptor α gene.

5. The method of claim 4, wherein antisense nucleotides, a ribozyme, a maxizyme, or an RNAi is used.

6. The method of claim 4, wherein DNA that encodes an antisense RNA, a ribozyme, a maxizyme, or an RNAi is used.

7. A substance for suppressing expression of PDGF receptor α comprising targeting mRNA containing exon 1 β among mRNAs of the PDGF receptor α gene.

8. The substance of claim 7, which is antisense nucleotides, a ribozyme, a maxizyme, or an RNAi.

9. The substance of claim 7, which is a DNA that encodes an antisense RNA, a ribozyme, a maxizyme, or an RNAi.

10. An agent for suppressing expression of PDGF receptor α comprising the substance of claim 7 as an active ingredient.

11. A therapeutic agent for cancer comprising the agent of claim 10.

12. A therapeutic method for cancer, wherein the agent of claim 10 is used.
